# EUROPEAN PATENT APPLICATION

(11) **EP 4 821 938 A1**
(43) Date of publication of application: **14.10.2026**
(21) Application number: 25169854.4
(22) Date of filing: 10.04.2025
(51) Int. Cl.: C07H 1/00, B01J 19/00, C07H 21/00

(54) **METHOD FOR THE PREPARATION OF DNA/RNA-OLIGO- AND POLYNUCLEOTIDES**

(71) Applicant: Universität Zürich, 8006 Zürich (CH); Novartis AG, 4056 Basel (CH)
(72) Inventor: Hartrampf, Nina, 8002 Zürich (CH); Lescure, Robin, 8135 Langnau am Albis (CH); Venturoni, Francesco, 68440 Schlierbach (CH); Haber, Julien, 4242 Laufen (CH); Naik, Anu, 4058 Basel (CH)
(74) Representative: Kasche & Partner

(57) **Abstract**

The present invention is directed to a method for the continuous flow preparation of oligonucleotide compounds using solid-phase flow chemistry implemented in two separated process chambers in continuous flow communication, wherein the first process chamber is an activation chamber (upstream chamber) for activating a nucleotide compound, and the second process chamber is a coupling chamber (downstream chamber) for coupling the activated nucleotide compound from the activation chamber to a solid-phase support (SPS)-bound mono-, di-, tri-, oligo- or polynucleotide compound, wherein both process chambers comprise a fluid-permeable divider for separating the activation chamber (a) from the coupling chamber (b) and its solid-phase bound mono-, di-, tri-, oligo- or polynucleotides.

The present invention further relates to an alternative method for the continuous flow preparation of oligonucleotide compounds using solid-phase flow chemistry, wherein the 5'-hydroxy of a mono-, di-, tri-, oligo- or polynucleotide bound to a solid phase support (SPS) is activated and reacted with the 3'-hydroxy of a nucleotide to prepare a di-, tri, oligo- or polynucleotide compound in continuous flow preparation.

## Description

The present invention is directed to a method for the continuous flow preparation of oligo- and polynucleotide compounds using solid-phase flow chemistry implemented in two separated process chambers in continuous flow communication, wherein the first process chamber is an activation chamber (upstream chamber) for activating a nucleotide compound, and the second process chamber is a coupling chamber (downstream chamber) for coupling the activated nucleotide compound from the activation chamber to a solid-phase support (SPS)-bound mono-, di-, tri-, oligo- or polynucleotide compound, wherein both process chambers comprise a fluid-permeable divider for separating the activation chamber (a) from the coupling chamber (b) and its solid-phase support-bound mono-, di-, tri-, oligo- or polynucleotides.

The present invention further relates to an alternative method for the continuous flow preparation of oligo- and polynucleotide compounds using solid-phase flow chemistry, wherein the 5'-hydroxy of a mono-, di-, tri-, oligo- or polynucleotide bound to a solid-phase support (SPS) is activated and reacted with the 3'-hydroxy of a nucleotide to prepare a di-, tri, oligo- or polynucleotide compound in continuous flow preparation.

Oligo- and polynucleotide therapeutics and diagnostics are currently on the rise, and an exceeding number of drugs recently received approval. While stability and delivery were a longstanding challenge, several chemical modifications significantly contributed to making oligonucleotide therapeutics more attractive, however, they were also accompanied with the requirement to produce those oligonucleotides chemically (most commonly via solid-phase synthesis, SPS) rather than using biological replication techniques.

With an increasing demand for chemically synthesized and modified RNA and DNA oligomers, their efficient and cost-effective production is currently an ongoing challenge in process chemistry.

Synthetic RNA and DNA polynucleotides are commonly prepared by the phosphoramidite strategy, and a typical synthesis cycle includes four steps: 1. deprotection (usually de-tritylation) of the 5'-terminal hydroxl group; 2. coupling of the nucleoside phosphoamidite, followed by 3. an optional capping step, and 4. an oxidation step of P(III) to P(V). These four steps are repeated until the length of the final oligonucleotide is reached, and a deprotection and cleavage is performed to release the desired oligonucleotide.

The phosphotriester approach (Phosphorous V, P(V)) was already published in the 1960s and would, in theory, consist of the same synthesis cycle, but would not require the oxidation step, thereby simplifying the overall process. Originally, this approach required a phosphate triester source and an activator and led to long reaction times with often low yields. Other and more activated phosphate sources, in particular dichlorophosphates, have been described by Letsinger et al. (Nucleic Acids Research, 1975, 6, 773-786) and Rizk et al. (Angew. Chem. Int. Ed., 1967, 8, 695-696). However, for these compounds rather low yields were observed, which was later attributed to the lack of activator and/or a catalyst. In 1976, Cashion et al. (Tetrahedron Letters, 1976, 42, 3769-3772) published a simplified one-pot method for the synthesis of DNA *via* the phosphotriester method with 1,2,4-triazole, chloro- or *N*-methylimidazole activation. The phosphorus agent had a *p*-chlorophenyl as a protecting group. This strategy led to the successful synthesis of dimers with yields up to 90 % and rather short reaction times (15-60 min.). It showed that a mixture of the dichlorophosphates with four equivalents of both 1,2,4-triazole and triethylamine and 16 equivalents of *N*-methylimidazole led to the best results.

Most commonly, the development of the phosphotriester method was carried out in the liquid phase. A few groups employed this method using solid-supported building blocks on PEG or glass supports (Lönnberg et al., Nucleic Acids Research, 1975, 2, 6, 773-786, Nucleic Acids Res. 1990, 18, 3155-3159, and Tetrahedron Letters, 1983, 24, 8, 747-750), which led to isolated yields for pentamers of around 55 %. However, this method had only been tested on 5-mers.

A few researchers investigated the combination of flow chemistry and oligonucleotide synthesis. The advantages of flow chemistry compared to batch chemistry are mainly an increased control over reaction parameters and an easier procedure, especially with iterative steps such as multiple synthetic cycles. Flow chemistry has already proven to be a powerful tool for peptide synthesis, making it easier, faster, and cleaner to produce long peptides of interest. The synthesis of oligonucleotides on a solid support using flow synthesizers is now very common. However, currently this method relies entirely on the phosphoramidite strategy and no attempt has been made for combining the phosphotriester strategy with flow chemistry.

It is the objective technical problem underlying the present invention to provide an improved method for preparing DNA and RNA oligo- and polynucleotides. The improvement is optionally the provision of reaction times comparable to those for standard phosphoramidite nucleotide coupling chemistry, the option for cheaper building blocks, i.e. more economic, avoiding the chemical oxidation of P(III) to P(V), and the optional use of so-called green solvents, e.g. 2-MeTHF, and high reaction yields, for example yields of about 90 %.

The above objective is solved by a method for the continuous flow preparation of oligo- and polynucleotide compounds according to reaction scheme
**(A)** comprising the steps of: 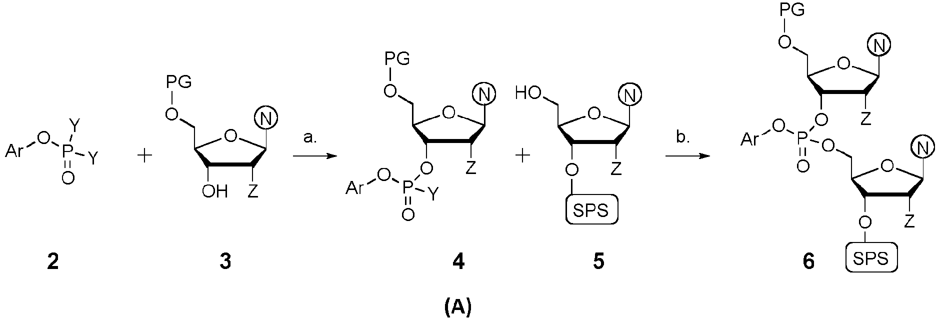
   a. reacting compound (2) with the 3'-hydroxy of a nucleotide (3) to prepare a 3'-activated nucleotide compound (4); and
   b. reacting the 3'-activated nucleotide compound (4) with the 5'-hydroxy of a solid-phase support (SPS)-bound mono-, di-, tri-, oligo- or polynucleotide (5) to prepare a di-, tri, oligo- or polynucleotide compound (6);
   wherein
   ∘ the further hydroxy group(s) in the nucleotide (3) and any amino groups in the base of nucleotide (3) are protected;
   ∘ **each N** is a protected nucleobase building block, wherein the nucleobase is independently selected from the group consisting of adenine (A), cytosine (C), guanine (G), thymine (T), uracil (U) and nucleobase derivatives thereof, optionally selected from the purine and pyrimidine group consisting of hypoxanthine, 2'-desoxyuracil, 4-thiouracil, fluorouracil, 8-azahypoxanthine, 8-azaadenine, allopurinol, 2-aminopurine, 2-amino-6-chloropurine , 2-amino-6-iodopurine, 8-bromotheo-phylline, xanthin, 1-methylxanthin, 3-methylxanthin, 7-methylxanthin, and 6-thioguanin;
   ∘ **each Z** is H, F, Br, Cl, N(R)₂, SR, or OR; wherein each R is independently selected from
      - substituted or non-substituted, branched or unbranched C₁-C₂₀, C₁-C₁₀, or C₁-C₆alkyl, alkene, aryl, ether, alkoxy, ester or acetal,
      - substituted or non-substituted, branched or unbranched C₁-C₂₀, C₁-C₁₀, or C₁-C₆ fluoroalkyl, chloroalkyl, bromoalkyl, aminoalkyl, guanidinoalkyl, cyanoalkyl,
   ∘ **Ar** is a substituted or non-substituted aromatic C₁-C₂₀, C₁-C₁₀, or C₄-C₆ carbocycle, optionally selected from phenyl and naphtyl carbocylces, methyl-, nitro-, F-, Cl-, Br- and NO₂-substituted carbocycles, optionally phenyl, mesityl, 2-chlorphenyl, 2,2-dichlorophenyl, 4-chlorophenyl, nitrophenyl, 2,2-dinitrophenyl, 4-nitrophenyl, 2-fluorophenyl, 2,2-difluorophenyl, 4-fluorophenyl, 2-iodophenyl, 2,2-diiodophenyl, 4-iodophenyl;
   ∘ **each Y** is selected from the group consisting of halides, optionally chloride, bromide and iodide; heterocyclic bases, optionally triazole and NMI; sulfonic acid derivatives, optionally mesyl, tosyl, brosyl, and triflyl; phosphonic acid; and phosphoric acid;
   ∘ **each PG** is an alcohol-protecting group, optionally selected from the group consisting of C₁-C₁₀, or C₁-C₆ esters, silyl ethers, benzyl ethers, alkyl ethers and acetals;
   ∘ **SPS** is a Solid-Phase Support, optionally selected from the group consisting of ceramic-, glass- or polymer-based solid-phase supports, optionally with a traceless chemically- or light-cleavable linker, optionally controlled pore glass (CPG), polystyrene resin (PS), polyethyleneglycol resin (PEG) or mixtures thereof, optionally attached to an ammonia-cleavable long-chain amino alkyl (LCAA), optionally CPG with a pore size of 500 Å and 0.3-0.4 mmol/g), optionally bound to a 4-amino-4-oxobutanoic ester linker,
   ∘ and the SPS is bound to nucleotide (5) by a 3'-hydroxy and the further hydroxy group(s) in the mono-, di-, tri-, oligo- or polynucleotide (5) and any amino groups in the base(s) of nucleotide(s) (5) are protected,
      characterized in that steps a. and b. are implemented in two separated process chambers in continuous flow communication,
      (a) an activation chamber (upstream chamber) for step a.,
      (b) a coupling chamber (downstream chamber) for step b., and
      (c) a fluid-permeable divider for separating the activation chamber (a) from the coupling chamber (b) and its solid-phase bound mono-, di-, tri-, oligo- or polynucleotides; or alternatively
**(B) comprising the steps of** 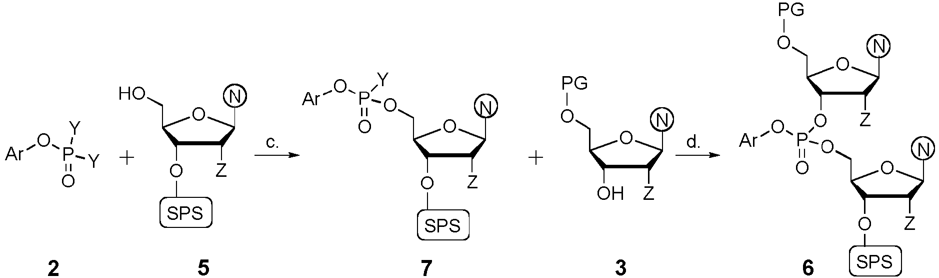
   c. reacting compound (2) with the 5'-hydroxy of a nucleotide bound to a solid phase support (SPS) (5), optionally bound to the solid phase support via further mono-, di-, tri-, oligo- or polynucleotides, to prepare a 5'-activated nucleotide compound (7);
   d. reacting the 5'-activated nucleotide compound (7) with the 3'-hydroxy of a nucleotide (3) to prepare a di-, tri, oligo- or polynucleotide compound (6);
   wherein
   the further hydroxy group(s) in the nucleotide and any amino groups in the base of nucleotide (5) are protected,
   the SPS is bound to nucleotide (5) by a 3'-hydroxy and the further hydroxy group(s) in the mono-, di-, tri-, oligo- or polynucleotide (5) and any amino groups in the base(s) of nucleotide(s) (5) are protected,
   characterized in that steps c. and d. are implemented in continuous flow communication.

The method of the present invention is directed to a new flow-based oligonucleotide synthesis method based on the phosphotriester P(V) strategy, which allows for using much cheaper building blocks than for the standard phosphoramidite method and which can be used for fast and economic continuous flow, avoids chemical oxidation from P(III) to P(V), and which is suitable for green solvents. The method demonstrated high coupling rates at room temperature with a minimum number of equivalents for the building block.

The method of the present invention is the first to combine the phosphotriester strategy based on a continuous approach. The method (A) is characterized in that that steps a. and b. are implemented in two separated process chambers in continuous flow communication,
(a) an activation chamber (upstream chamber) for step a.,
(b) a coupling chamber (downstream chamber) for step b., and
(c) a fluid-permeable divider for separating the activation chamber (a) from the coupling chamber (b) and its solid-phase bound mono-, di-, tri-, oligo- or polynucleotides.

In method (A) of the invention the residence time in the activation chamber (a) is adjusted so that a substantial part, optionally most of the activation reactants 2 and 3, optionally at least 50, 60, 70, 80 or 90 % of the activation reactants, result in activated nucleotide 4 before reaching the coupling chamber via the fluid permeable divider in order to avoid any undesired cross reactions between the activation compounds 2 and 3 and the coupling compounds 4 and 5.

In method (B) of the invention the activation step of reactants 2 and 5 and the coupling step of reactants 7 and 3 may be performed in one or two connected process chambers. Because activation reactant 5 is SPS (solid-phase support)-bound, alternative flows of activation reactant 2 and coupling reactant 3 will lead to continuous coupling of the nucleotide reactant(s) 3 to produce SPS-bound di-, tri, oligo- or polynucleotides. The continuous flow of soluble reactants with the stably supported and sequentially activated SPS-nucleotide avoids undesired cross reaction of activation and coupling reactants. Flow and residence time in method (B) of the invention are adjusted to optimize reaction yield of the activation and coupling of the SPS-bound reactant 5 and 6.

Both, methods of the invention (A) and (B) result in SPS-bound di-, tri-, oligo- or polynucleotides 6 depending on the number of cycles with activation and coupling of coupling reactants 5 (A) or 3 (B).

It is obvious that for several cycles of method A the intermediate and final product compounds 6 must be cleaved from the SPS for generating the free 3'-hydroxyl of compound 3 for use in a further activation and coupling cycle. The resulting phosphorous ester linkage between coupled nucleotides in product compounds 6 may be converted to a common nucleotide phosphate linkage either before each activation step or when releasing the finished di, tri, oligo of polynucleotide product. (European Journal of Organic Chemistry, 2013, 35, 7886-7890. For the final coupling product of method A the final compound 6 may be cleaved from the SPS and the protecting group at the 5'-hydroxyl may be removed by conventional de-protection chemistry (Organic & Biomolecular Chemistry, 2005, 3, 21, 3851-3868*.*) (Current Protocols in Nucleic Acid Chemistry, 2013, 3.1.1-3.1.60)*.* Hence, in one alternative, the method A of the present invention further comprises at least one of the following steps of
- cleaving the SPS from compound 6,
- deprotecting the 5'-hydroxyl and/or
- transforming the aryl phosphate into phosphate.

For several cycles of method B the intermediate and final product compounds 6 must be deprotected at the 5'-hydroxyl for generating the free 5'-hydroxyl of compound 5 for use in a further activation and coupling cycle. The phosphorous ester linkage between coupled nucleotides in product compounds 6 may be converted to a common nucleotide phosphate linkage either before each activation step or when releasing the finished di-, tri-, oligo- or polynucleotide product (see European Journal of Organic Chemistry, 2013, 35, 7886-7890). For the final coupling product of method B the final compound 6 may be cleaved from the SPS and the protecting group at the 5'-hydroxyl may be removed by conventional de-protection chemistry. Hence, in one alternative, the method B of the present invention further comprises at least one of the following steps of
- cleaving the SPS from compound 6,
- deprotecting the 5'-hydroxyl, and/or
- transforming the aryl phosphate into phosphate.

The terms nucleotide, activated and/or protected nucleotide, oligo- and polynucleotide, as used herein, are meant to encompass single nucleotides, chemically activated nucleotides for the chemical addition of nucleotides to other nucleotides, protected nucleotides for nucleotide chain addition, wherein selected substituents of the nucleotide(s) are chemically protected to avoid undesired side reactions, to form RNA, DNA, and PNA with two to ten (oligonucleotides) nucleotide bases, and more than 10, optionally 10 to 10000, 10 to 1000, 10 to 100, 10 to 50, 10 to 30 nucleotide bases (polynucleotides).

The nucleotide bases N for practicing the present invention are protected nucleobase building blocks and encompass functional derivatives and analogs, i.e. compounds which may function as nucleotide bases for adenine A, thymine T, guanin G, cytosin C, and uracil U, however with chemical derivatization. Optionally, the method of the invention is one, wherein the nucleotide bases N of the nucleotides for use in the inventive method are each selected from the group consisting of *N*(6)-benzoyl adenine (A), *N*(6)-isobutyryl adenine (A), *N*(6)-phenoxyacetyl adenine (A), *N*(4)-benzoyl cytosine (C), (4)-acetyl cytosine (C), *N*(4)-isobutyril guanine (G), *N*(4)-isopropyl-phenoxyacetyl guanine (G), *N*(4)-dimethylformamidino guanine (G), thymine (T), uracil (U), hypoxanthine, 2'-desoxyuracil, 4-thiouracil, fluorouracil, 8-azahypoxanthine, *N*(6)-benzoyl-8-azaadenine, *N*(6)-isobutyryl-8-azaadenine, *N*(6)-phenoxyacetyl 8-azaadenine, allopurinol, *N*(2)-benzoyl-2-aminopurine, *N*(2)-isobutyryl-2-aminopurine, *N*(2)- phenoxyacetyl-2-aminopurine, *N*(2)-benzoyl-2-amino-6-chloropurine, *N*(2)- isobutyryl -2-amino-6-chloropurine, *N*(2)-phenoxyacetyl-2-amino-6-chloropurine, *N*(2)-benzoyl-2-amino-6-iodopurine, *N*(2)-isobutyryl-2-amino-6-iodopurine, *N*(2)- phenoxyacetyl-2-amino-6-iodopurine, 8-bromotheophylline, xanthin, 1-methylxanthin, 3-methylxanthin, 7-methylxanthin, *S*(6)-(*p*-acetyloxybenzyl)-thioguanin.

Further suitable optional nucleobases for practicing the invention include but are not limited to modified nucleobases, such as xanthine, hypoxanthine, 2-aminoadenine, 6-methyl and other alkyl derivatives of adenine and guanine, 2-propyl and other alkyl derivatives of adenine and guanine, 5-halo uracil and cytosine, 6-azo uracil, cytosine and thymine, 5-uracil (pseudo uracil), 4-thiouracil, 8-halo, oxa, amino, thiol, thioalkyl, hydroxyl and other 8-substituted adenines and guanines, 5-trifluoromethyl and other 5-substituted uracils and cytosines, 7-methyl-guanine. Further naturally- and non-naturally-occurring nucleobases include those disclosed in U.S. Pat. No. 3,687,808 (Merigan et al.); in Sanghvi, in Antisense Research and Application, Chapter 15, S. T. Crooke and B. Lebleu, Eds., CRC Press, 1993; in Englisch et al., Angewandte Chemie International Edition, 1991, 30, 613-722 (particularly, pages 622 and 623); in the Concise Encyclopedia of Polymer Science and Engineering, J. I. Kroschwitz, Ed., John Wiley & Sons, 1990, pages 858-859; in Zhang, et al., Nature, 2017, 551, 644-647 (hydrophobic bases); in Feldman and Romesberg, Acc. Chem. Res. 2018, 51, 394-403; and in Cook, Anti-Cancer Drug Design, 1991, 6, 585-607, each of which is hereby incorporated by reference in its entirety.

Further suitable nucleobases for practicing the present invention are specifically listed in US2023/212204A1, which document is referenced in its entirety.

The term "continuous flow preparation", as used herein, is meant to indicate that at least the activation and coupling steps a. to d. of method A and B are implemented in a continuous flow of solvent(s) and reactants with a residence time suitable for substantial transformation of the reactants into intermediate and final products. Optionally, SPS-cleavage, deprotection, rinse, purification, etc. practiced in the context of the present invention may also be implemented as continuous flow reactions or processes.

In the method of the present invention substituent Z optionally represents a protected 2'-hydroxyl of nucleotides 3 to 7, optionally selected from the group consisting of methoxy, ethoxy, phenyl, trifluoromethyl, aminoethyl, guanidinoethyl, cyanoethyl acetyl, benzoyl, pivaloyl, trimethylsilyl, triethylsilyl, *tert*-butyldimethylsilyl, triisopropylsilyl, *tert*-butyldiphenylsilyl, benzyl, trityl, p-methoxybenzyl, dimethoxytrityl, *p*-pethoxyphenyl, allyl, *t*-butyl, methoxymethyl, tetrahydropyranyl, (triisopropylsilyl)oxy]-methyl, 2-nitrobenzyl)oxy]methyl, bis(acetoxyethoxy)methyl, *t*-butyldithiomethyl, etrahydro-4-methoxy-2H-pyran-2-yl, 1-(2-cyanoethoxy)ethyl, 2-cyanoethoxymethyl, 4-(*N*-dichloroacetyl-N-methylamino)benzyloxy-methyl.

In an alternative embodiment, the method of the present invention is one, wherein **Ar-P(O)₂Y** is selected from the group of consisting of phenyl dichlorophosphate, mesityl dichlorophosphate, 2-chlorophenyl , phenyl dichlorophosphate, mesityl dichlorophosphate, 2-chlorophenyl phosphorodichloridate, 2,2-dichlorophenyl phosphorodichloridate, 4-chlorophenyl phosphorodichloridate, 2-nitrophenyl phosphorodichloridate, 2,2-dinitrophenyl phosphorodichloridate 4-nitrophenyl phosphorodichloridate, 2-fluorophenyl phosphorodichloridate, 2,2-difluorophenyl phosphorodichloridate, 4-fluorophenyl phosphorodichloridate, 2-iodophenyl phosphorodichloridate, 2,2-diiodophenyl phosphorodichloridate, 4-iodophenyl phosphorodichloridate.

The protecting group PG of the 5'-hydroxy in nucleotides 3, 4, and 6 is optionally independently selected for each nucleotide from the group consisting of trityl, methoxytrityl, dimethoxytrityl, diethoxytrityl , trimethoxytrityl, 9-phenyl xanthen-9-yl, 9-(*p*-tolyl)-xanthen-9-yl, 4,4',4"-tris(benzoyloxy)trityl, 4-dinitrobenzenesulfenyl, acetyl, benzoyl, pivaloyl, trimethylsilyl, triethylsilyl, *tert*-butyldimethylsilyl, triisopropylsilyl, *tert*-butyldiphenylsilyl, benzyl, p-methoxybenzyl, dimethoxytrityl, *p*-methoxyphenyl, allyl, *t*-butyl, methoxymethyl, and tetrahydropyranyl.

In an optional embodiment of the present invention each Y in compound 2 is independently selected from the group consisting of halides, heterocyclic bases, sulphonic acid derivatives, phosphonic acid derivatives and/or phosphoric acid derivatives; optionally compound 2 is selected from the group consisting of chloride, bromide, iodide, triazole, alkylimidazole, imidazole, mesyl, tosyl, brosyl, triflyl, alkyl phosphonic acids, aryl phosphonic acids, trialkylphosphoric acid and triarylphosphoric acid, wherein alkyl and aryl are optionally C1 to 20, optionally C1 to 10 or C1 to 6, optionally branched or unbranched, substituted or unsubstituted.

The optimum residence time of the reactants in the process chamber(s) for practicing the methods A and B of the present invention depends on the flow rate and the dimensions of the chamber(s). An optimum flow rate and residence time depends on the reactivity and the concentration of the reactants as well as the solvent(s), the temperature, activating and catalytic co-compounds and can be can be determined without undue burden by routine experimentation simply varying parameters. For example, the flow rate of activation chamber (a) and coupling chamber (b) may be adjusted so that a substantial part, optionally most of the activation reactants 2 and 3, optionally at least 50, 60, 70, 80 or 90 % of the activation reactants result in activated nucleotide 4 before reaching the coupling chamber via the fluid permeable divider in order to avoid any undesired cross reactions between the activation compound 2 and 3 and the coupling compounds 4 and 5.

Optionally, the activation steps a. and c. of the present invention are implemented in the presence of one or more catalysts selected from 1,2,4-triazole about 4 equivalents TEA (triethylamine), and about 16 equivalents NMI (N-Methylimidazole).

Furthermore, anhydrous solvents such as 2-MeTHF and/or THF and/or pyridine and/or toluene and/or methanol and/or acetonitrile, are preferred for practicing the methods of the present invention.

The terms "activation chamber" and "coupling chamber", as used herein, refer to separate chambers, i.e. reactors, reaction volumes/vessels, connected via a fluid-permeable divider, for example a glass, ceramic or polymer frit, a polymer sheet, retaining any solids, in particular SPS-bound nucleotides, but fluid permeable for solvent(s) and soluble reactants and intermediate products. The chambers can be made of any shaped material resistant to chemical damage by the solvents and reactants, e.g. glass, ceramic, polymers, etc. Optionally, a stirrer, e.g. a mechanical or magnetic stirrer, within the one or two chambers can be used to further mix reactants during continuous flow operations.

The two chambers may be positioned adjacent to each other or on top of each other. For example, a most simple design would be a cylinder with two chambers separated by a frit for retaining solid materials and allowing for a flow in vertical direction.

In the context of the present invention, it is understood that antecedent terms such as "linear or branched", "substituted or non-substituted" indicate that each one of the subsequent terms is to be interpreted as being modified by said antecedent term. For example, the scope of the term "linear or branched, substituted or non-substituted alkyl, alkenyl, alkynyl, carbocycle" encompasses linear or branched, substituted or non-substituted alkyl; linear or branched, substituted or non-substituted alkenyl; linear or branched, substituted or non-substituted alkynyl; linear or branched, substituted or non-substituted alkylidene; and linear or branched, substituted or non-substituted carbocycle. For example, the term "(C2-10) alkenyl, alkynyl or alkylidene" indicates the group of compounds having 2 to 10 carbons and alkenyl, alkynyl or alkylidene functionality.

The expression "alkyl" refers to a saturated, straight-chain or branched hydrocarbon group that contains the number of carbon items indicated, e.g. "(C1-10)alkyl" denotes a hydrocarbon residue containing from 1 to 10 carbon atoms, e.g. a methyl, ethyl, propyl, iso-propyl, n-butyl, iso-butyl, sec-butyl, tert-butyl, n-pentyl, iso-pentyl, n-hexyl, 2,2-dimethylbutyl, etc.

The expression "alkenyl" refers to an at least partially unsaturated, substituted or non-substituted straight-chain or branched hydrocarbon group that contains the number of carbon atoms indicated, e.g. "(C2-10)alkenyl" denotes a hydrocarbon residue containing from 2 to 10 carbon atoms, for example an ethenyl (vinyl), propenyl (allyl), iso-propenyl, butenyl, isoprenyl or hex-2-enyl group, or, for example, a hydrocarbon group comprising a methylene chain interrupted by one double bond as, for example, found in monounsaturated fatty acids or a hydrocarbon group comprising methylene-interrupted polyenes, e.g. hydrocarbon groups comprising two or more of the following structural unit -[CH=CH-CH2]-, as, for example, found in polyunsaturated fatty acids. Alkenyl groups have one or more, preferably 1, 2, 3, 4, 5, or 6 double bond(s).

The expression "alkynyl" refers to at least partially unsaturated, substituted or non-substituted straight-chain or branched hydrocarbon groups that contain the number of carbon items indicated, e.g. "(C₂₋₁₀)alkynyl" denotes a hydrocarbon residue containing from 2 to 10 carbon atoms, for example an ethinyl, propinyl, butinyl, acetylenyl, or propargyl group. Preferably, alkynyl groups have one or two (especially preferably one) triple bond(s).

Furthermore, the terms "alkyl", "alkenyl" and "alkynyl" refer to groups in which one or more hydrogen atom(s) have been replaced, e.g. by a halogen atom, preferably F or Cl, such as, for example, a 2,2,2-trichloroethyl or a trifluoromethyl group.

The term "carbocycle" shall be understood to mean a substituted or non-substituted hydrocarbon cycle containing the number of carbon items indicated, e.g. "(C₃₋₁₀)carbocycle" or from 3 to 20, preferably from 3 to 12 carbon atoms, more preferably 5 or 6 carbon atoms. These carbocycles may be either aromatic or non-aromatic systems. The non-aromatic ring systems may be mono- or polyunsaturated.

The term "carbobicycle" refers to a carbocycle as defined above comprising more than 1 ring, preferably two rings. Preferred carbocycles and carbobicycles include but are not limited to cyclopropyl, cyclobutyl, cyclopentyl, cyclopentenyl, cyclohexyl, cyclohexenyl, cycloheptanyl, cycloheptenyl, phenyl, indanyl, indenyl, benzocyclobutanyl, dihydronaphthyl, tetrahydronaphthyl, naphthyl, decahydronaphthyl, benzocycloheptanyl, benzocycloheptenyl, spiro[4,5]decanyl, norbornyl, decalinyl, bicyclo[4.3.0]nonyl, tetraline, or cyclopentylcyclohexyl. The carbo- and/or carbobicyclic residue may be bound to the remaining structure of the complete molecule by any atom of the cycle, which results in a stable structure

The term "carbocycle" shall also include "cycloalkyl" which is to be understood to mean aliphatic hydrocarbon-containing rings preferably having from 3 to 12 carbon atoms. These non-aromatic ring systems may be mono- or polyunsaturated, i.e. the term encompasses cycloalkenyl and cycloalkynyl.

The term "heterocycle" refers to a stable substituted or non-substituted, aromatic or non-aromatic, preferably 3 to 20 membered, more preferably 3-12 membered, most preferably 5 or 6 membered, monocyclic, heteroatom-containing cycle. Each heterocycle consists of carbon atoms and one or more, preferably 1 to 4, more preferably 1 to 3 heteroatoms preferably chosen from nitrogen, oxygen and sulphur. A heterocycle may contain the number of carbon atoms in addition to the non-carbon atoms as indicated: a "(C₃₋₆)heterocycle" is meant to have 3 to 6 carbon atoms in addition to a given number of heteroatoms.

In the following the present invention is illustrated by way of representative examples, none of which are to be construed as limiting the scope of the invention beyond the appended claims.

### Figures

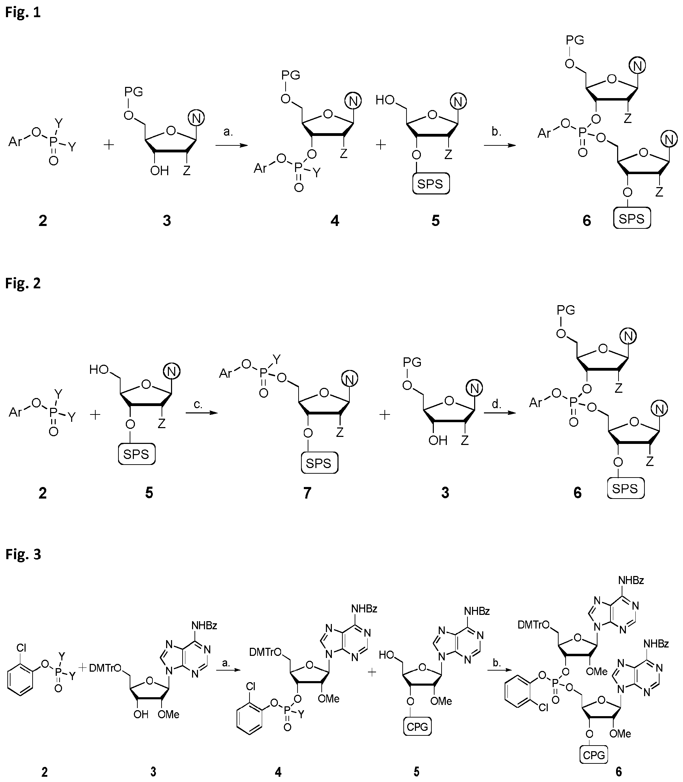

**Fig. 1** illustrates reaction scheme A: activation of secondary alcohol 3 and coupling to immobilized primary alcohol 5 to yield compound **6.** N = protected and unprotected purine and pyrimidine bases (=A, G, C, T, U + N-derivatives and analogs). Y = leaving group (= chloride, triazole, NMI), Ar = aryl group.
   Reaction conditions: a. 2-chlorophenyl phosphorodichloridate (0.9-14.4 eq), anhydrous solvent (e.g. 2-MeTHF, toluene), elevated temperatures (40-90 °C), residence times: 2-10 min; b. Solid-supported alcohol (0.02-0.2 eq.), anhydrous solvent (e.g. 2-MeTHF, toluene), elevated temperatures (40-90 °C), 10-20 min.
**Fig. 2** illustrates reaction scheme B: activation of immobilized primary alcohol 5 and coupling to secondary alcohol **3** to yield compound **6.** N = protected and unprotected purine and pyrimidine bases (=A, G, C, T, U + N-derivatives and analogs). Y = leaving group (= chloride, triazole, NMI), Ar = aryl group.
   Reaction conditions: c. anhydrous solvent (e.g. 2-MeTHF, toluene), elevated temperatures (40-90 °C), 2-10 min; d. anhydrous solvent (e.g. 2-MeTHF, toluene), elevated temperatures (40-90 °C), 10-60 min.
**Fig. 3** illustrates an specific example of Reaction scheme A: activation of secondary alcohol 3 and coupling to immobilized primary alcohol 5 to yield compound 6. Y = leaving group (=triazole, NMI). Reaction conditions: a. anhydrous 2-MeTHF, 75 °C, residence time: 4.8 min; b. anhydrous 2-MeTHF, 75 °C, 16.2 min.

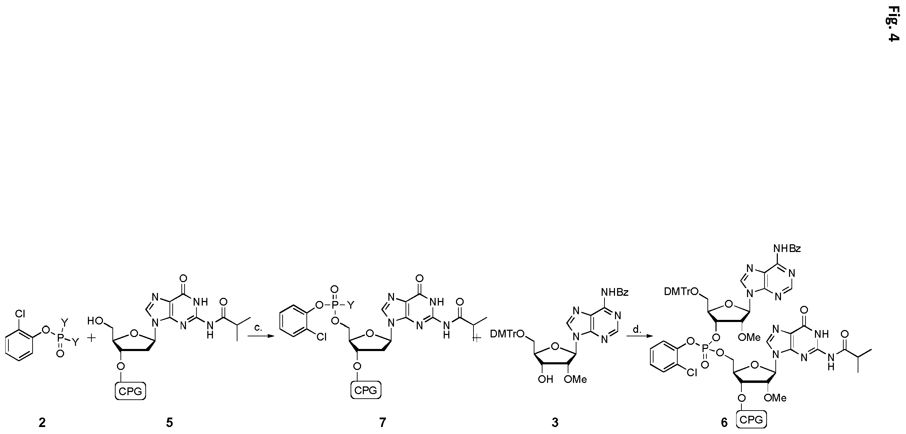

**Fig. 4****:** Optional example for Reaction scheme B: activation of primary immobilized alcohol 5 and coupling to secondary alcohol **3** to yield compound **6.** Y = leaving group (=triazole, NMI). Preferred reaction conditions: a. anhydrous 2-MeTHF, 60 °C, residence time: 7.5 min; b. anhydrous 2-MeTHF, 60 °C, 7.5 min.

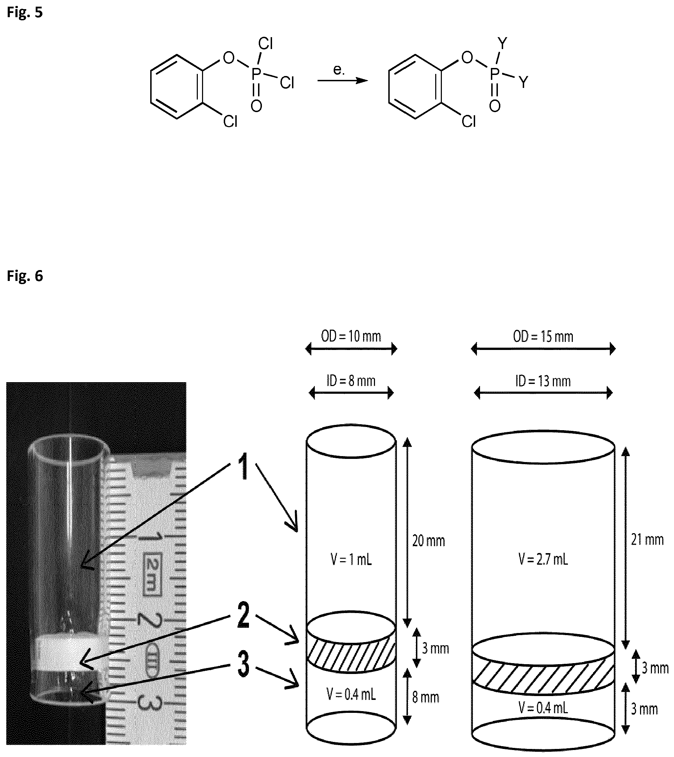

**Fig. 5** shows the reaction scheme of the batch preactivation step. Reaction conditions for the activation of 2-chlorophenyl phosphorodichloridate 1 are modified from Cashion *et al.* (10.1016/- S0040-4039(00)93105-8). Reaction conditions: e. Triazole (16 eq.), TEA (14 eq.), NMI (139 eq.), anhydrous 2-MeTHF, 20-75 °C, 15-60 min. Optional conditions: a. Triazole (4 eq.), TEA (4 eq.), NMI (16 eq.), anhydrous 2-MeTHF, 23 °C, 20 min.
**Fig. 6** shows a two-chamber glas reactor with coupling chamber (**1**) / frit (**2**) / activation chamber (**3**);

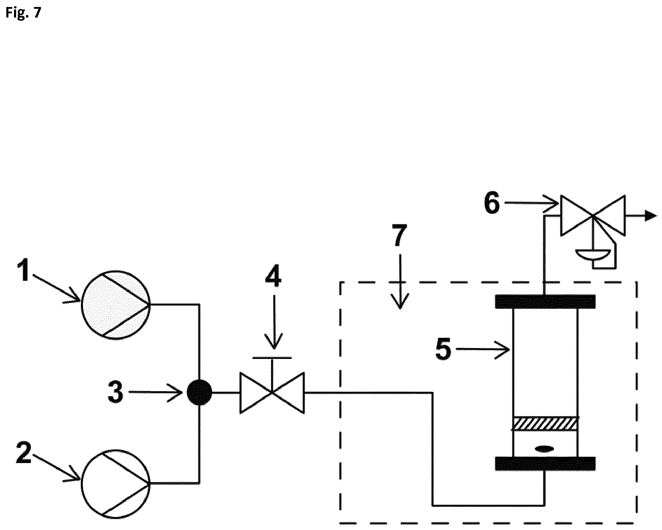

**Fig 7** is a schematic drawing of a **r**eaction set up for the coupling step. Syringe pump **1** transfers the building block solution and syringe pump **2** transfers the activated phosphorous agent solute-ion (Qₜₒₜ = 5 mL/h). A T-piece (**3**) is used to connect the two incoming reagent lines. A finger valve (**4**) is placed immediately after the T-piece (**3**). The finger valve is connected via tubing with the inlet of the reactor (**5**, shown in Fig. 6). The outlet of the reactor (**5**) is connected to a backpressure regulator (**6**, p = 50 psi) that leads to the waste.

### Examples

### Example 1- Preactivation Step

1,2,4-triazole (117 mg, 1.68 mmol, 2.74 eq.), 2-MeTHF (3.30 mL), triethylamine (206 µL, 1.47 mmol, 2.40 eq.) and *N*-methylimidazole (1.13 mL, 14.2 mmol, 23.2 eq.) were added to a 10 mL Schlenk reactor fitted with a stirring bar (both dried overnight at 200 °C) under argon atmosphere. 1.03 mL (613 µmol) of a solution of 2-chlorophenyl phosphorodichloridate (**1**, 183 uL) in 2-MeTHF (1.71 mL) was added to the Schlenk flask, instantly forming a bright yellow precipitate. The mixture was stirred at 1500 rpm at 23 °C for 20 min. The resulting yellow suspension was then filtered over Spherose filters (Nylon 0.22 um, Ø 13 mm) in a dried conical flask (dried at 200 °C overnight) under argon atmosphere.

Due to the instability of activated compound **2,** no purification and further characterization were carried out, and compound **2** was immediately used in the next step.

### Example 2 - Reaction scheme A

Coupling: A reaction setup as illustrated in **Fig. 7** was built, and the reactor with immobilized compound **5** was placed in a water bath at 75 °C. A 0.18 M solution of the oligonucleotide building block **3** in 2-MeTHF was prepared in a 10 mL-Schlenk reactor fitted with a stirring bar under an argon atmosphere. The clear solution with oligonucleotide building block (**3**, 1.9 mL) was transferred into a Luer lock 2.5 mL-syringe. The filtered activated phosphorous agent (**2**, 1.9 mL) was transferred into another Luer lock 2.5 mL-syringe (**Fig. 7**). Both syringes were placed in a syringe pump, and the flow rate was set to 5 mL/h and ran for 45 min.

Wash: The syringes were exchanged to 5 mL-Luer lock syringes with 2-MeTHF, and the pumps were run at 5 mL/h for an additional 80 min. The pump was then run at a flow rate of 2 mL/min for 3 min. After letting the reactor cool to 23 °C, two 5 mL-Luer lock syringes containing dichloromethane were installed and the pump ran at 2 mL/min for 6 min min.

Deprotection: On-flow deprotection was carried out with trichloroacetic acid (3% in dichloromethane, 2x2.5 mL) at a flow rate of 2 mL/min. A washing step with anhydrous dichloromethane (2x6 mL) was performed at a flow rate of 2 mL/min for 6 min. A second washing step with anhydrous 2-MeTHF (2x6 mL) was performed at a flow rate of 2 mL/min for 5 min. The reactor was placed back in the water bath at 75 °C, and another washing step with anhydrous 2-MeTHF (2 mL) was performed at a flow rate of 10 mL/h.

Crude purity: 90% as identified by uHPLC and LC-MS.

### Example 3 - Reaction scheme B

Phosphorous agent solution preparation: To a Schlenk tube, 1,2,4-triazole (84.0 mg, 1.22 mmol, 100 eq.), then anhydrous THF (6 mL), then triethylamine (150 uL, 1.10 mmol, 86 eq.), then *N-*methylimidazole (850 uL, 4.9 mmol, 5.9 eq.), and finally a solution of 2-chlorophenyl phosphorodichloridate (**1**,120 uL, 0.80 mmol, 64 eq.) in THF (2 mL) were added. The mixture was stirred at 23 °C for 20 min.

On-support activation: Immobilized oligonucleotide building block **5** was placed in the reactor and rinsed with anhydrous THF (20 mL) at a flow rate of 8 mL/min. Afterwards, the reactor temperature was raised to 60 °C. The filtered phosphorous agent solution **2** was transferred into a Luer lock 2.5 mL-syringe, and a syringe pump connected to the reactor transferred compound 2 over building block **5** with a flow rate of 8 mL/h and for 60 min.

Wash: The syringes were replaced with 10 mL-Luer lock syringes with anhydrous 2-MeTHF, and the pumps ran at a flow rate of 8 mL/h for an additional 15 min.

Coupling: The second building block (**3**, 360 mg, 0.530 mmol, 43 eq.) in THF (8 mL) was placed in a Schlenk tube. The mixture was then transferred in a 10 mL-Luer lock syringe. The syringe pump was set to a flowrate of 8 mL/h.

Wash: The syringes were replaced with 10 mL-Luer lock syringes with anhydrous 2-MeTHF, and the pumps ran at a flow rate of 100 mL/h. The syringes were exchanged to 10 mL-Luer lock syringes with dichloromethane, and the pumps ran at a flow rate of 100 mL/h.

Deprotection: On-flow deprotection was carried out with trichloroacetic acid (3% in dichloromethane, 2x2.5 mL) at a flow rate of 2 mL/min. A washing step with anhydrous dichloromethane (2x6 mL) was performed at a flow rate of 2 mL/min for 6 min. A second washing step with anhydrous 2-MeTHF (2x6 mL) was performed at a flow rate of 2 mL/min for 5 min. The reactor was placed back in the water bath at 75 °C, and another washing step with anhydrous 2-MeTHF (2 mL) was performed at a flow rate of 10 mL/h.

### Example 4 - Cleavage from the solid support

The solid support was placed in a Falcon tube and an ammonium hydroxide solution (25-30% NH₃ basis, 1 mL) was added. The Falcon tube was tightly sealed and placed on a vortex shaker at 800 rpm and 55 °C for 2 h. The tube was then carefully opened, and aqueous phosphate buffer (0.05 M, 5 mL) was added. The supernatant was then filtered over Spherose filters, and the sample was directly analyzed by LC-MS and uHPLC analysis.

## Claims

1. A method for the continuous flow preparation of oligo- and polynucleotide compounds according to reaction scheme
**(A) comprising the steps of:** 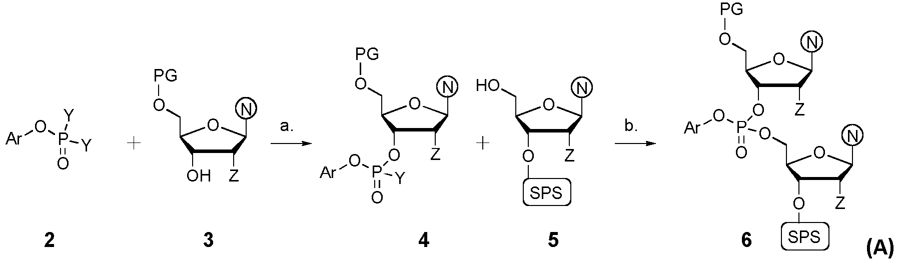
a. reacting compound (2) with the 3'-hydroxy of a nucleotide (3) to prepare a 3'-activated nucleotide compound (4); and
b. reacting the 3'-activated nucleotide compound (4) with the 5'-hydroxy of a solid phase support (SPS)-bound mono-, di-, tri-, oligo- or polynucleotide (5) to prepare a di-, tri, oligo- or polynucleotide compound (6);
wherein
∘ the further hydroxy group(s) in the nucleotide (3) and any amino groups in the base of nucleotide (3) are protected;
∘ **each N** is a protected nucleobase building block, wherein the nucleobase is independently selected from the group consisting of adenine (A), cytosine (C), guanine (G), thymine (T), uracil (U) and nucleobase derivatives thereof, optionally selected from the purine and pyrimidine group consisting of hypoxanthine, 2'-desoxyuracil, 4-thiouracil, fluorouracil, 8-azahypoxanthine, 8-azaadenine, allopurinol, 2-aminopurine, 2-amino-6-chloropurine , 2-amino-6-iodopurine, 8-bromotheophylline, xanthin, 1-methylxanthin, 3-methylxanthin, 7-methylxanthin, and 6-thioguanin;
∘ **each Z** is H, F, Br, Cl, N(R)₂, SR, or OR; wherein each R is independently selected from
- substituted or non-substituted, branched or unbranched C₁-C₂₀, C₁-C₁₀, or C₁-C₆ alkyl, alkene, aryl, ether, alkoxy, ester or acetal,
- substituted or non-substituted, branched or unbranched C₁-C₂₀, C₁-C₁₀, or C₁-C₆ fluoroalkyl, chloroalkyl, bromoalkyl, aminoalkyl, guanidinoalkyl, cyanoalkyl,
∘ **Ar** is a substituted or non-substituted aromatic C₁-C₂₀, C₁-C₁₀, or C₄-C₆ carbocycle, optionally selected from phenyl and naphtyl carbocylces, methyl-, nitro-, F-, Cl-, Br- and NO₂-substituted carbocycles, optionally phenyl, mesityl, 2-chlorphenyl, 2,2-dichlorophenyl, 4-chlorophenyl, nitrophenyl, 2,2-dinitrophenyl, 4-nitrophenyl, 2-fluorophenyl, 2,2-difluorophenyl, 4-fluorophenyl, 2-iodophenyl, 2,2-diiodophenyl, 4-iodophenyl;
∘ **each Y** is selected from the group consisting of halides, optionally chloride, bromide and iodide; heterocyclic bases, optionally triazole and NMI; sulfonic acid derivatives, optionally mesyl, tosyl, brosyl, and triflyl; phosphonic acid; and phosphoric acid;
∘ **each PG** is an alcohol-protecting group, optionally selected from the group consisting of C₁-C₁₀, or C₁-C₆ esters, silyl ethers, benzyl ethers, alkyl ethers and acetals;
∘ **SPS** is a Solid Phase Support, optionally selected from the group consisting of ceramic-, glass- or polymer-based solid phase supports, optionally with a traceless cleavable linker, optionally controlled pore glass (CPG), polystyrene resin (PS), polyethyleneglycol resin (PEG) or mixtures thereof, optionally attached to a chemically- or light-cleavable linker, optionally CPG with a pore size of 500 Å and 0.3-0.4 mmol/g, optionally bound to an ammonia-cleavable C1-? amino alkyl linker,
∘ and the SPS is bound to nucleotide (5) by a 3'-hydroxy and the further hydroxy group(s) in the mono-, di-, tri-, oligo- or polynucleotide (5) and any amino groups in the base(s) of nucleotide(s) (5) are protected,
**characterized in that** steps a. and b. are implemented in two separated process chambers in continuous flow communication,
(d) an activation chamber (upstream chamber) for step a.,
(e) a coupling chamber (downstream chamber) for step b., and
(f) a fluid-permeable divider for separating the activation chamber (a) from the coupling chamber (b) and its solid-phase bound mono-, di-, tri-, oligo- or polynucleotides; or
**(B) comprising the steps of** 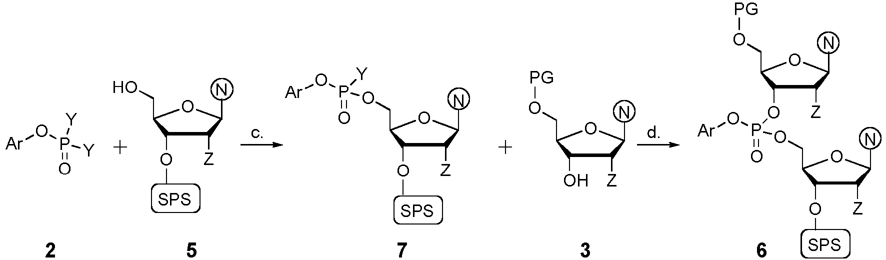
c. reacting compound (2) with the 5'-hydroxy of a nucleotide bound to a solid phase support (SPS) (5), optionally bound to the solid phase support via further mono-, di-, tri-, oligo- or polynucleotides, to prepare a 5'-activated nucleotide compound (7);
d. reacting the 5'-activated nucleotide compound (7) with the 3'-hydroxy of a nucleotide (3) to prepare a di-, tri, oligo- or polynucleotide compound (6);
wherein
the further hydroxy group(s) in the nucleotide and any amino groups in the base of nucleotide (5) are protected,
the SPS is bound to nucleotide (5) by a 3'-hydroxy and the further hydroxy group(s) in the mono-, di-, tri-, oligo- or polynucleotide (5) and any amino groups in the base(s) of nucleotide(s) (5) are protected,
**characterized in that** steps c. and d. are implemented in continuous flow communication.

2. The method of claim 1, wherein method A or method B further comprises at least one of the following steps of
- cleaving the SPS from compound 6,
- deprotecting the 5'-hydroxyl, and/or
- transforming the aryl phosphate into phosphate.

3. The method of any of claims 1 or 2, wherein the nucleotides N are each selected from the group consisting of N(6)-benzoyl adenine (A), N(6)-isobutyryl adenine (A), N(6)-phenoxyacetyl adenine (A), N(4)-benzoyl cytosine (C), (4)-acetyl cytosine (C), N(4)-isobutyril guanine (G), *N*(4)-isopropylphenoxyacetyl guanine (G), *N*(4)-dimethylformamidino guanine (G), thymine (T), uracil (U), hypoxanthine, 2'-desoxyuracil, 4-thiouracil, fluorouracil, 8-azahypoxanthine, *N*(6)-benzoyl-8-azaadenine, *N*(6)-isobutyryl-8-azaadenine, *N*(6)-phenoxyacetyl 8-azaadenine, allopurinol, *N*(2)-benzoyl-2-aminopurine, *N*(2)-isobutyryl-2-aminopurine, *N*(2)-phenoxyacetyl-2-aminopurine, *N*(2)-benzoyl-2-amino-6-chloropurine, *N*(2)- isobutyryl -2-amino-6-chloropurine,*N*(2)-phenoxyacetyl-2-amino-6-chloropurine, *N*(2)-benzoyl-2-amino-6-iodopurine, *N*(2)-isobutyryl-2-amino-6-iodopurine, *N*(2)- phenoxyacetyl-2-amino-6-iodopurine, 8-bromotheophylline, xanthin, 1-methylxanthin, 3-methylxanthin, 7-methylxanthin, *S*(6)-(*p*-acetyloxybenzyl)-thioguanin.

4. The method of any of claims 1 to 3, wherein **Z** is selected from the group consisting of methoxy, ethoxy, phenyl, trifluoromethyl, aminoethyl, guanidinoethyl, cyanoethyl acetyl, benzoyl, pivaloyl, trimethylsilyl, triethylsilyl, *tert*-butyldimethylsilyl, triisopropylsilyl, *tert*-butyldiphenylsilyl, benzyl, trityl, p-methoxybenzyl, dimethoxytrityl, *p*-pethoxyphenyl, allyl, *t*-butyl, methoxymethyl, tetrahydropyranyl, (triisopropylsilyl)oxy]-methyl, 2-nitrobenzyl)oxy]methyl, bis(acetoxyethoxy)methyl, *t*-butyldithiomethyl, etrahydro-4-methoxy-2H-pyran-2-yl, 1-(2-cyanoethoxy)ethyl, 2-cyanoethoxymethyl, 4-(*N*-dichloroacetyl-N-methylamino)benzyloxy-methyl.

5. The method of any one of claims 1 to 4, wherein Ar-P(O)₂Y is selected from the group of consisting of phenyl dichlorophosphate, mesityl dichlorophosphate, 2-chlorophenyl, phenyl dichlorophosphate, mesityl dichlorophosphate, 2-chlorophenyl phosphorodichloridate, 2,2-dichlorophenyl phosphorodichloridate, 4-chlorophenyl phosphorodichloridate, 2-nitrophenyl phosphorodichloridate, 2,2-dinitrophenyl phosphorodichloridate 4-nitrophenyl phosphorodichloridate, 2-fluorophenyl phosphorodichloridate, 2,2-difluorophenyl phosphorodichloridate, 4-fluorophenyl phosphorodichloridate, 2-iodophenyl phosphorodichloridate, 2,2-diiodophenyl phosphorodichloridate, 4-iodophenyl phosphorodichloridate.

6. The method of any one of claims 1 to 5, wherein each PG is independently selected from the group consisting of trityl, methoxytrityl, dimethoxytrityl, diethoxytrityl , trimethoxytrityl, 9-phenyl xanthen-9-yl, 9-(*p*-tolyl)-xanthen-9-yl, 4,4',4"-tris(benzoyloxy)trityl, 4-dinitrobenzenesulfenyl, acetyl, benzoyl, pivaloyl, trimethylsilyl, triethylsilyl, *tert*-butyldimethylsilyl, triisopropylsilyl, *tert*-butyldiphenylsilyl, benzyl, p-methoxybenzyl, dimethoxytrityl, *p-*methoxyphenyl, allyl, *t*-butyl, methoxymethyl, and tetrahydropyranyl.

7. The method of any one of claims 1 to 6, wherein each Y in compound 2 is independently selected from the group consisting of halides, heterocyclic bases, sulphonic acid derivatives, phosphonic acid derivatives and/or phosphoric acid derivatives; optionally compound 2 is selected from the group consisting of chloride, bromide, iodide, triazole, alkylimidazole, imidazole, mesyl, tosyl, brosyl, triflyl, alkyl phosphonic acids, aryl phosphonic acids, trialkylphosphoric acid and triarylphosphoric acid.

8. The method of any one of claims 1 to 7, wherein activation steps a. and c. are implement-ted in the presence of one or more catalysts selected from 1,2,4-triazole about 4 equivalents TEA (triethylamine), and about 16 equivalents NMI (N-Methylimidazole).
